(19) **Europäisches Patentamt**

**European Patent Office**

**Office européen des brevets**

(11) Veröffentlichungsnummer: **0 127 766**
**B1**

(12) # EUROPÄISCHE PATENTSCHRIFT

(45) Veröffentlichungstag der Patentschrift:
**19.02.86**

(51) Int. Cl.⁴: **C 07 C 69/54,** C 07 C 67/08,
C 07 C 67/26

(21) Anmeldenummer: **84104854.9**

(22) Anmeldetag: **30.04.84**

(54) **Verfahren zur Herstellung von (Meth)Acrylsäureestern und deren Verwendung.**

(30) Priorität: **06.05.83 DE 3316592**

(43) Veröffentlichungstag der Anmeldung:
**12.12.84 Patentblatt 84/50**

(45) Bekanntmachung des Hinweises auf die Patenterteilung:
**19.02.86 Patentblatt 86/8**

(84) Benannte Vertragsstaaten:
**BE CH DE FR GB IT LI NL SE**

(56) Entgegenhaltungen:
**EP - A - 0 029 709**
**EP - A - 0 057 834**
**DE - A - 2 758 019**
**US - A - 4 207 155**

(73) Patentinhaber: **BASF Aktiengesellschaft,
Carl-Bosch-Strasse 38, D-6700 Ludwigshafen (DE)**

(72) Erfinder: **Schornick, Gunnar, Dr.,
Konrad-Adenauer-Strasse 8, D-6719 Neuleiningen (DE)**
Erfinder: **Buethe, Ingolf, Dr., Am Wasserturm 1,
D-6737 Boehl-Iggelheim (DE)**
Erfinder: **Jacobi, Manfred, Dr., Heidelberger Ring 32 b,
D-6710 Frankenthal (DE)**
Erfinder: **Lenz, Werner, Dr.,
Heinrich-Baermann-Strasse 14, D-6702 Bad Duerkheim
(DE)**
Erfinder: **Straehle, Wolfgang, Dr., Hirschgasse 20,
D-6900 Heidelberg (DE)**
Erfinder: **Krause, Hans-Joachim, Dr., Pariser Strasse 31,
D-6700 Ludwigshafen (DE)**

## Beschreibung

Die vorliegende Erfindung betrifft ein Verfahren zur Herstellung von (Meth)Acrylsäureestern geringer Farbzahl durch Veresterung von (Meth)-Acrylsäure mit ein- bzw. mehrwertigen, im allgemeinen bis 4wertigen Alkoholen und deren Verwendung in Formulierungen, die durch UV- und Elektronenstrahlen schnell gehärtet werden können.

(Meth)Acrylsäureester mono- und polyfunktioneller Alkohole werden vorzugsweise als Reaktivverdünner in strahlungshärtbaren Bindemittel-Formulierungen eingesetzt. Gefordert sind dabei farblose Produkte mit geringer Säurezahl. Die Herstellung solcher (Meth)Acrylsäureverbindungen ist in der Literatur beschrieben. Am häufigsten werden sie durch direkte Veresterung von (Meth)-Acrylsäure mit den entsprechenden OH-Komponenten unter Verwendung eines sauren Veresterungskatalysators in Gegenwart eines aliphatischen, cycloaliphatischen oder aromatischen Kohlenwasserstoffs als Schleppmittel hergestellt (vgl. z.B. DE-AS Nr. 2020419, DE-AS Nr. 2647890 oder US-PS Nr. 4207155). Die Aufarbeitung der auf diese Weise erhaltenen Reaktionsmischungen erfolgt durch Waschen der organischen Phase mit Wasser bzw. wässerigen Lösungen oder durch Vakuumdestillation. Werden die (Meth)Acrylsäureverbindungen in einen Umesterungsschritt unter Katalysierung mit Metallen bzw. deren Verbindungen oder mit organischen Säuren hergestellt, ist ebenfalls ein Destillationsschritt erforderlich (vgl. z. B. DE-OS Nr. 2637409 und DE-OS Nr. 2939001).

Allen beschriebenen Herstellverfahren ist die Tatsache gemeinsam, dass das Veresterungsgemisch einem Aufarbeitungsschritt unterzogen werden muss. Abgesehen vom reinen zeitlichen Aufwand für Auswaschvorgang und/oder Destillation sind diese Herstellverfahren mit Ausbeuteverlusten verbunden, die je nach Polyol-Typ zwischen 5 und 20% betragen können.

Ziel der vorliegenden Erfindung war es, strahlungshärtbare Reaktivverdünner für strahlungshärtbare Überzugsmassen so herzustellen, dass die eingesetzten Rohstoffe praktisch ohne Ausbeuteverluste zu den entsprechenden Endprodukten umgesetzt werden, ohne dass Eigenschaften wie Farbzahl oder Reaktivität der damit hergestellten Bindemittelformulierung nachteilig beeinflusst werden.

Die Aufgabe wurde dadurch gelöst, dass die nach der Umsetzung der (Meth)Acrylsäure mit der OH-Komponente im Veresterungsgemisch vorhandene Rest(meth)acrylsäure stöchiometrisch mit einem Epoxid umgesetzt wird.

Gegenstand der vorliegenden Erfindung ist ein Verfahren zur Herstellung von Estern der Acryl- oder Methacrylsäure mit hydroxylgruppenhaltigen organischen Verbindungen, das dadurch gekennzeichnet ist, dass man gesättigte, gegebenenfalls Ethergruppen enthaltende Alkohole, die, sofern sie 2 oder mehr freie Hydroxylgruppen pro Molekül enthalten, Molekulargewichte zwischen 62 und 399 aufweisen und, sofern sie nur eine freie Hydroxylgruppe pro Molekül enthalten, ein Molekulargewicht zwischen 130 und 300 aufweisen, mit 100 bis 150 Mol-%, bezogen auf die OH-Gruppen des Alkohols, Acrylsäure oder Methacrylsäure in Gegenwart eines sauren Veresterungskatalysators und mindestens eines Kohlenwasserstoffs, der mit Wasser ein azeotropes Gemisch bildet, sowie geringer Mengen eines Polymerisationsinhibitors unter azeotroper Entfernung des entstehenden Wassers bei erhöhter Temperatur verestert, nach der Veresterung den Kohlenwasserstoff destillativ entfernt und nach Neutralisation des Veresterungskatalysators die restliche Acryl- oder Methacrylsäure mit einer der Säurezahl äquivalenten Menge einer Epoxidverbindung bis zu einer Säurezahl ⩽ 5 mg KOH/g umsetzt, mit der Massgabe, dass, sofern Alkohole mit 2 oder mehr freien Hydroxylgruppen pro Molekül mit Acrylsäure oder Methacrylsäure verestert wurden, als Epoxidverbindungen solche mit mindestens 2 Epoxidgruppen pro Molekül verwendet werden, sofern jedoch Alkohole mit nur einer freien Hydroxylgruppe pro Molekül mit Acrylsäure oder Methacrylsäure verestert wurden, als Epoxidverbindungen Monoepoxidverbindungen verwendet werden.

Bevorzugt ist die Verwendung von 1,2-Epoxybutan als Monoepoxidverbindung, bzw. als Epoxidverbindung mit mindestens 2 Epoxidgruppen pro Molekül die der Glycidylether des Bisphenols A, des Butandiols und des Pentaerythrits.

Bevorzugt ist ausserdem, das erfindungsgemässe Verfahren so durchzuführen, dass das Umsetzungsprodukt der restlichen Acrylsäure oder Methacrylsäure mit der Epoxidverbindung in einer Menge von 5 bis 25 Gew.-% der Gesamtmenge des Veresterungsproduktgemisches erhalten wird.

Gegenstand der vorliegenden Erfindung ist ausserdem die Verwendung der nach dem erfindungsgemässen Verfahren hergestellten Produkte als Reaktivverdünner in strahlungshärtbaren Überzugsmassen.

Zu den für das erfindungsgemässe Verfahren zu verwendenden einzelnen Komponenten ist folgendes auszuführen.

Als mit Acryl- bzw. Methacrylsäure zu veresternde Alkohole, die 2 oder mehr freie Hydroxylgruppen pro Molekül enthalten und Molekulargewichte zwischen 62 und 399 aufweisen, eignen sich beispielsweise Diole, wie Ethylenglykol, Propylenglykol, Butandiol-1,4, Pentandiol-1,5, Neopentylglykol, Hexandiol-1,6, 2-Methylpentandiol-1,5, 2-Ethylbutandiol-1,4, Dimethylolcyclohexan, 1,1'-Isopropyliden-bis-(p-phenylenoxy)-di-β-ethanol (= ®Dianol 22), Triole, wie Glycerin, Trimethylolethan, Trimethylolpropan, Trimethylolbutan, Tetraole, wie Pentaerythrit sowie Oxethylierungs- und Oxpropylierungsprodukte der vorstehend genannten Alkohole und von Bisphenol A mit 1 bis 10 mol Ethylenoxid oder Propylenoxid pro Mol OH-Gruppen.

Als mit Acryl- bzw. Methacrylsäure zu veresternde Alkohole mit nur einer freien Hydroxylgruppe pro Molekül, die Molekulargewichte zwischen 130 und 300 aufweisen, kommen Monoalkohole mit 8 bis 20 Kohlenstoffatomen in Frage, beispielsweise 2-Ethylhexanol, Laurylalkohole, Stearylalkohol, 4-tert.-Butylcyclohexanol, 3,3,5-Trimethylcyclohexanol, 2-Methyl-3-phenylpropanol-1, Phenylglykol sowie Oxethylierungs- und Oxpropylierungsprodukte von 2 bis 12 Kohlenstoffatome enthaltenden Monoalkoholen mit 1 bis 5 mol Ethylenoxid bzw. Propylenoxid.

Die Alkohole werden mit 100 bis 150 Mol-%, vorzugsweise 110 bis 125 Mol-%, bezogen auf die OH-Gruppen des Alkohols, Acrylsäure oder Methacrylsäure in Gegenwart eines sauren Veresterungskatalysators und eines Kohlenwasserstoffs, der mit Wasser ein azeotropes Gemisch bildet, verestert.

Als saure Veresterungskatalysatoren dienen handelsübliche anorganische oder organische Säuren oder saure Ionenaustauscher, wobei Schwefelsäure und p-Toluolsulfonsäure bevorzugt sind. Sie werden im allgemeinen in Mengen von 0,1 bis 3 Gew.-%, bezogen auf das zu veresternde Gemisch aus (Meth)acrylsäure und Alkohol eingesetzt.

Die Veresterung erfolgt unter azeotropen Bedingungen bei erhöhter Temperatur, im allgemeinen bei 40 bis 120°C, vorzugsweise bei 70 bis 115°C, wobei sich als Schleppmittel zur Entfernung des Reaktionswassers Kohlenwasserstoffe, wie aliphatische, cycloaliphatische oder aromatische Kohlenwasserstoffe oder Gemische derselben, vorzusweise solche mit einem Siedebereich zwischen 70 und 120°C als besonders geeignet erwiesen haben.

Geeignete aliphatische Kohlenwasserstoffe sind z. B. Hexan und seine Isomeren sowie Kohlenwasserstoffgemische mit Siedebereich zwischen 60 und 95°C.

Besonders bevorzugte Schleppmittel sind Cyclohexan und Toluol. Die Menge des zugesetzten Kohlenwasserstoffs ist unkritisch; je nach verwendeter Apparatur kann die zugesetzte Menge zwischen der 0,1- und 2fachen Menge des Reaktionsgemisches aus (Meth)Acrylsäure und Alkohol variieren. Besonders vorteilhaft ist ein Verhältnis Reaktionsgemisch zu Kohlenwasserstoff von 1:0,20 bis 1:0,8.

Die OH-Komponenten und (Meth)Acrylsäure werden in Äquivalentverhältnissen von 1:1 bis 1:1,5 eingesetzt, d. h. auf ein Grammäquivalent OH des Alkohols kommen 1 bis 1,5 Grammäquivalent (Meth)Acrylsäure, bevorzugt ist ein Äquivalentverhältnis von 1:1 bis 1:1,25.

An die mitzuverwendenden Polymerisationsinhibitoren werden in Hinblick auf Verfärbung des Reaktionsgemisches besondere Anforderungen gestellt, da bei dem erfindungsgemässen Herstellverfahren das Veresterungsgemisch weder durch Auswaschvorgänge noch durch Vakuumdestillation aufgearbeitet wird. So sind beispielsweise die sehr gut wirksamen Cu-Verbindungen (DE-OS Nr. 2838691), die ebenfalls gut wirksamen aromatischen Nitro- und N-Nitrosoverbindungen (DE-OS Nr. 2449054, DE-OS Nr. 2502530, DE-OS Nr. 2502954) nicht einsetzbar, da sie in den Acrylestern hohe Farbzahlen verursachen. Als geeignet haben sich erwiesen Inhibitoren auf Basis ein- und mehrwertiger Phenole wie beispielsweise p-Methoxyphenol, 2,6-Di-tert.-butyl-p-Kresol, Hydrochinonmonomethylether, Resorcinmonomethylether, ausserdem Verbindungen vom Typ des Thiodiphenylamins (= Phenothiazin) und Zinn-IIverbindungen. Vorteilhaft sind Kombinationen aus Zinn-II- und Phenolverbindung sowie die Kombination Zinn-II-verbindung + Phenolverbindung + Phenothiazin. Die Verbindungen auf Phenolbasis werden vorzugsweise in Mengen von 0,05 bis 1%, die Zinn-II-verbindung in Mengen von 0,01 bis 1% Sn und Phenothiazin in Mengen von 0,001 bis 0,1% eingesetzt, jeweils bezogen auf das Veresterungsgemisch aus OH-Komponente und (Meth)-Acrylsäure.

Ein wesentlicher Vorteil des erfindungsgemässen Veresterungsverfahrens besteht darin, dass auf Waschvorgänge zur Entfernung des Veresterungskatalysators und nicht umgesetzter (Meth)Acrylsäure verzichtet wird und dass der Destillationsschritt lediglich zur Abtrennung des Schleppmittels dient. Nach beendeter Auskreisung des Reaktionswassers wird das Schleppmittel abdestilliert, der Veresterungskatalysator mit der äquivalenten Menge einer Base, z. B. eines tertiären Amins oder eines Alkali- bzw. Erdalkalihydroxids oder des entsprechenden Carbonats neutralisiert.

Dann wird der Reaktionsmischung eine der Säurezahl äquivalente Menge einer Epoxidverbindung zugesetzt, wobei für den Fall, dass Alkohole mit nur einer freien Hydroxylgruppe pro Molekül mit Acrylsäure oder Methacrylsäure verestert wurden, als Epoxidverbindung eine Monoepoxidverbindung, wie z. B. Versaticsäureglycidylester und Alkyl- bzw. Arylglycidylether, wie n-Butylglycidylether, 2-Ethylhexylglycidylether, Phenylglycidylether, o-Kresylglycidylether oder vorzugsweise 1,2-Epoxybutan, eingesetzt wird; für den Fall, dass Alkohole, die 2 oder mehr freie Hydroxylgruppen pro Molekül enthalten, mit Acrylsäure oder Methacrylsäure verestert wurden, werden als Epoxidverbindung solche mit mindestens 2 Epoxidgruppen eingesetzt, beispielsweise di- oder trifunktionelle Epoxide, wie di- oder trifunktionelle Glycidylether oder -erster. Bevorzugt sind Polyglycidylverbindungen vom Bisphenol-A-Typ oder Glycidylether mehrfunktioneller Alkohole, wie Butandiol, Glycerin oder Pentaerythrit. Beispiele für derartige Polyglycidylverbindungen sind Bisphenol-A-diglycidylether wie ® Epikote 828 (Epoxidwert: ca. 0,53), Butandioldiglycidylether und Pentaerythrittriglycidylether.

Die Umsetzung mit den Epoxidverbindungen erfolgt vorzugsweise in Gegenwart von Katalysatoren, wie z. B. Thiodiglykol bei Temperaturen von 80 bis 130°C, vorzugsweise bei 95 bis 115°C.

Bei der erfindungsgemässen Herstellungsweise werden die Einsatzstoffe praktisch quantitativ ohne Ausbeuteverluste zum Endprodukt umgesetzt.

Ausserdem ist durch das erfindungsgemässe Verfahren gewährleistet, dass die entstehenden Produkte sehr kleine Säurezahlen aufweisen und somit ihr Einsatz in mit UV- und Elektronenstrahlen härtbaren Bindemittelformulierungen in keiner Weise beschränkt ist. Sie können überall dort Verwendung finden, wo auch bisher Acryl- und Methacrylsäureester der oben genannten Alkohole als Reaktivverdünner und/oder Vernetzer eingesetzt werden. Bevorzugt ist die Verwendung der erfindungsgemäss hergestellten Produkte als Reaktivverdünner in strahlungshärtbaren Überzugsmassen und im Falle der von monofunktionellen Alkoholen ausgehenden Acrylester als Comonomere in Mischungen aus olefinisch ungesättigten Verbindungen.

Die Härtung mittels UV-Strahlen erfolgt im allgemeinen in Gegenwart üblicher UV-Initiatoren, wie z. B. Benzoinethern, Benzilketalen, Acylphosphinoxiden, Acetophenon-Derivaten, die im allgemeinen in Mengen von 0,1 bis 10%, vorzugsweise 0,5 bis 4 Gew.-%, bezogen auf polymerisierbare Verbindungen eingesetzt werden. Geeignete Strahlungsquellen sind z. B. Mitteldruckquecksilberdampfstrahler, wie sie etwa auch in „UV Curing: Science and Technology" (Hrsg. S.P. Pappas) beschrieben werden.

Die Härtung kann jedoch auch mittels ionisierender Strahlung, beispielsweise mittels Elektronenstrahlen bei Beschleunigungsspannungen von 150 bis 500 kV erfolgen.

Die in den Beispielen angegebenen Teile und Prozente sind, soweit nicht ausdrücklich anders angegeben, Gewichtsteile und Gewichtsprozente.

*Beispiel 1:*

OH-Komponente, Acrylsäure, Schleppmittel, Veresterungskatalysator und Inhibitoren werden in den in Tabelle 1 angegebenen Mengen zusammengegeben und aufgeheizt. Unter Auskreisung des gebildeten Reaktionswassers wird die Veresterung bei 80°C durchgeführt. Sobald die berechnete Wassermenge abdestilliert ist, wird das Schleppmittel abdestilliert und die Säurezahl des Veresterungsgemisches ermittelt. Nach Neutralisation des Veresterungskatalysators wird die der Säurezahl äquivalente Epoxidmenge zugesetzt und nach Zugabe des Katalysators bei 105 bis 110°C umgesetzt, bis eine Endsäurezahl < 5 erreicht ist.

*Tabelle 1*

| | | | | | | |
|---|---|---|---|---|---|---|
| Acrylsäure | (Teile) | 842 | 842 | 842 | 950 | 1125 |
| Alkoxyliertes Trimethylolpropan (OHZ = 525) | (Teile) | 1000 | 1000 | 1000 | — | — |
| Hexandiol-1,6 | (Teile) | — | — | — | 708 | — |
| Trimethylolpropan | | — | — | — | — | 558 |
| Cyclohexan | (Teile) | 921 | 921 | 921 | 850 | 850 |
| Hydrochinonmonomethylether | (Teile) | 5,5 | 5,5 | 3,75 | 3,4 | 3,4 |
| Phenothiazin | (Teile) | 0,1 | 0,1 | — | 0,1 | 0,1 |
| $SnCl_2 \cdot H_2O$ | (Teile) | — | — | 3,6 | 3,2 | 3,2 |
| Schwefelsäure 96%ig | (Teile) | 6 | 6 | 6 | 17,5 | 17,5 |
| SZ-Veresterungsgemisch (mg KOH/g) | (Teile) | 39 | 40,4 | 40 | 29 | 34 |
| Dimethylethanolamin | (Teile) | 11,2 | 11,2 | 11,2 | 57,2* | 57,2* |
| Thiodiglykol | (Teile) | 2,5 | 2,5 | 2,5 | 15 | |
| Epoxid | (Teile) | 175 (I) | 159 (II) | 155 (III) | 105 (III) | 113 (III) |
| SZ-Produkt (mg KOH/g) | | 1,9 | 1,1 | 3,0 | 4,1 | 3,8 |
| $\eta$ 23°C (mPas) | | 425 | 275 | 195 | | |
| Auslaufzeit DIN 4 (s) | | | | | 12 | 26 |
| * wässerige NaOH-Lsg. (25%ig) | | | | | | |

I = ®Epikote 828
II = Pentaerythrittriglycidylether
III = Butandioldiglycidylether

*Beispiele 2 und 3:*

Anstelle der mehrfunktionellen Alkohole in Beispiel 1 wurden als OH-Verbindung Monoalkohole eingesetzt. Die Komponenten wurden in den nachstehend aufgeführten Mengen zusammengegeben und aufgeheizt. Nach Erreichen einer Innentemperatur von 95 bis 100°C wurden innerhalb von 7½ h die angegebenen Wassermengen ausgekreist. Das Lösungsmittel wurde zunächst bei Normaldruck, dann im Vakuum bei ca. 50 mbar abgezogen. Anschliessend wurden das Amin, die monofunktionelle Epoxidverbindung und der Katalysator zugesetzt. Die weitere Umsetzung wurde bei ca. 105°C bis zum Erreichen einer Säurezahl < 5 mg KOH/g durchgeführt.

| | Beispiel 2 | Beispiel 3 |
|---|---|---|
| Phenylglykol | 620 | — |
| Decalol-2 | — | 910 |
| Acrylsäure | 395 | 511 |
| Cyclohexan | 300 | 490 |
| Hydrochinonmonomethylether | 1 | 0,3 |
| 2,6-Di-tert.-butyl-p-Kresol | 1 | 1,3 |
| Thiodiphenylamin | 0,01 | — |
| Hypophosphorige Säure | — | 0,5 |
| Schwefelsäure | 2,5 | — |
| p-Toluolsulfonsäure | — | 28 |
| Ausgekreiste Wassermenge (ml) | 93 | 133 |
| SZ (mg KOH/g) | 42 | 17 |
| Dimethylethanolamin | 4,8 | 11 |
| o-Kresylglycidylether | 117 | — |
| Versaticsäureglycidylester | — | 122 |
| Thiodiglykol | 1,8 | 1,8 |
| SZ (mg KOH/g) | 2,6 | 4,1 |
| η 23° C (mPas) | 10 | 45 |

## Patentansprüche

1. Verfahren zur Herstellung von Estern der Acryl- oder Methacrylsäure mit hydroxylgruppenhaltigen organischen Verbindungen, dadurch gekennzeichnet, dass man gesättigte, gegebenenfalls Ethergruppen enthaltende Alkohole, die, sofern sie 2 oder mehr freie Hydroxylgruppen pro Molekül enthalten, Molekulargewichte zwischen 62 und 399 aufweisen und, sofern sie nur eine freie Hydroxylgruppe pro Molekül enthalten, ein Molekulargewicht zwischen 130 und 300 aufweisen, mit 100 bis 150 Mol-%, bezogen auf die OH-Gruppen des Alkohols, Acrylsäure oder Methacrylsäure in Gegenwart eines sauren Veresterungskatalysators und mindestens eines Kohlenwasserstoffs, der mit Wasser ein azeotropes Gemisch bildet, sowie geringer Mengen eines Polymerisationsinhibitors unter azeotroper Entfernung des entstehenden Wassers bei erhöhter Temperatur verestert, nach der Veresterung den Kohlenwasserstoff destillativ entfernt und nach Neutralisation des Veresterungskatalysators die restliche Acryl- oder Methacrylsäure mit einer der Säurezahl äquivalenten Menge einer Epoxidverbindung bis zu einer Säurezahl ≤ 5 mg KOH/g umsetzt, mit der Massgabe, dass, sofern Alkohole mit 2 oder mehr freien Hydroxylgruppen pro Molekül mit Acrylsäure oder Methacrylsäure verestert wurden, als Epoxidverbindungen solche mit mindestens 2 Epoxidgruppen pro Molekül verwendet werden, sofern jedoch Alkohole mit nur einer freien Hydroxylgruppe pro Molekül mit Acrylsäure oder Methacrylsäure verestert wurden, als Epoxidverbindungen Monoepoxidverbindungen verwendet werden.

2. Verfahren nach Anspruch 1, dadurch gekennzeichnet, dass als Monoepoxidverbindung 1,2-Epoxybutan verwendet wird.

3. Verfahren nach Anspruch 1, dadurch gekennzeichnet, dass als Epoxidverbindung mit mindestens 2 Epoxidgruppen pro Molekül der Triglycidylether des Pentaerythrits verwendet wird.

4. Verfahren nach Anspruch 1, dadurch gekennzeichnet, dass als Polyepoxidverbindung Glycidylether vom Bisphenol-A-Typ eingesetzt werden.

5. Verfahren nach Anspruch 1, dadurch gekennzeichnet, dass als Polyepoxidverbindung der Glycidylether des Butandiols-1,4 eingesetzt wird.

6. Verfahren nach einem der vorhergehenden Ansprüche, dadurch gekennzeichnet, dass das Umsetzungsprodukt der restlichen Acrylsäure oder Methacrylsäure mit der Epoxidverbindung in einer Menge von 5 bis 25 Gew.-% der Gesamtmenge des Veresterungsproduktgemisches erhalten wird.

7. Verwendung der nach einem Verfahren gemäss einem der vorhergehenden Ansprüche hergestellten Produkte als Reaktivverdünner in strahlungshärtbaren Überzugsmassen.

## Claims

1. A process for the preparation of esters of acrylic or methacrylic acid with hydroxyl-containing organic compounds, wherein saturated alcohols, which optionally contain ether groups and which, if they contain 2 or more free hydroxyl groups per molecule, have a molecular weight of from 62 to 399 and, if they contain only one free hydroxyl group per molecule, have a molecular weight of from 130 to 300, are esterified at elevated temperature with from 100 to 150 mole%, based on the OH groups of the alcohol, of acrylic or methacrylic acid in the presence of an acidic esterification datalyst and at least one hydrocarbon which forms an azeotropic mixture with water, and of small amounts of a polymerization inhibitor, with the azeotropic removal of the water formed; following the esterification, the hydrocarbon is removed by distillation; and after neutralization of the esterification catalyst, the remaining acrylic or methacrylic acid is reacted with an epoxy compound having an acid number of ≤ 5 mg KOH/g in an amount equivalent to the acid number, with the proviso that, if alcohols containing 2 or more free hydroxyl groups per molecule have been esterified with acrylic or methacrylic acid, epoxy compounds are used which contain at least 2 epoxy groups per molecule, and that, if alcohols containing only one free hydroxyl group per molecule have been esterified with acrylic or methacrylic acid, monoepoxy compounds are used as epoxy compounds.

2. A process as claimed in Claim 1, wherein 1,2-epoxybutane is used as monoepoxy compound.

3. A process as claimed in Claim 1, wherein the triglycidyl ether of pentaerythritol is used as epoxy compound containing at least 2 epoxy groups per molecule.

4. A process as claimed in Claim 1, wherein glycidyl ethers of bisphenol A are used as polyepoxy compounds.

5. A process as claimed in Claim 1, wherein the glycidyl ether of 1,4-butanediol is used as polyepoxy compound.

6. A process as claimed in any of the preceding claims, wherein the reaction product of the remaining acrylic or methacrylic acid with the epoxy compound is obtained in an amount of from 5 to 25% by weight, based on the total amount of the esterification product mixture.

7. The use of the products prepared by a process as claimed in any of the preceding claims as reactive diluents in radiation-curable coating compositions.

## Revendications

1. Procédé de préparation d'esters d'acide acrylique ou méthacrylique, avec des composés organiques à groupes hydroxyle, caractérisé par le fait que l'on estérifie, à température élevée, des alcools saturés, contenant éventuellement des groupes éther, et qui, dans la mesure où ils contiennent 2 ou plus de 2 groupes hydroxyle libres par molécule, présentent des poids moléculaires compris entre 62 et 399, et, dans la mesure où ils ne contiennent qu'un groupe hydroxyle libre par molécule, présentent un poids moléculaire compris entre 130 et 300, avec 100 à 150 mol-%, rapporté aux groupes OH de l'alcool, d'acide acrylique ou méthacrylique, en présence d'un catalyseur acide d'estérification et d'au moins un hydrocarbure qui forme avec l'eau un mélange azéotrope, ainsi que de petites quantités d'un inhibiteur de polymérisation, avec élimination azéotrope de l'eau produite, on élimine par distillation l'hydrocarbure, après l'estérification et, après neutralisation du catalyseur d'estérification, on fait réagir l'acide acrylique ou méthacrylique restant avec une quantité équivalente à l'indice d'acide d'un composé époxy jusqu'à un indice d'acide ≤ 5 mg KOH/g, sous réserve que, dans la mesure où ce sont des alcools ayant 2 ou plus de deux groupes hydroxyle libres par molécule, qui ont été estérifiés avec de l'acide acrylique ou méthacrylique, on utilise, comme composés époxy, ceux ayant au moins 2 groupes époxy par molécule et, dans la mesure où ce sont des alcools n'ayant qu'un groupe hydroxyle libre par molécule qui ont été estérifiés avec de l'acide acrylique ou méthacrylique, on utilise, comme composés époxy, des composés monoépoxy.

2. Procédé selon la revendication 1, caractérisé par le fait que, comme composé monoépoxy, on utilise le 1,2-époxybutane.

3. Procédé selon la revendication 1, caractérisé par le fait que, comme composé époxy ayant au moins 2 groupes époxy par molécule, on utilise l'éther triglycidylique du pentaérythrite.

4. Procédé selon la revendication 1, caractérisé par le fait que, comme composé polyépoxyde, on introduit de l'éther glycidylique du bisphénol type A.

5. Procédé selon la revendication 1, caractérisé par le fait que, comme composé polyépoxy, on introduit l'éther glycidylique du butanediol-1,4.

6. Procédé selon l'une des revendications précédentes, caractérisé par le fait que le produit de réaction de l'acide acrylique ou méthacrylique restant avec le composé époxy est obtenu en quantité de 5 à 25% en poids de la quantité totale du mélange de produit de réaction.

7. Utilisation des produits obtenus par un procédé selon l'une des revendications précédentes comme diluants réactifs dans des masses de revêtement durcissables aux rayonnements.